(19) Europäisches Patentamt
European Patent Office
Office européen des brevets

(11) Veröffentlichungsnummer: **0 282 908**
**A2**

# (12) EUROPÄISCHE PATENTANMELDUNG

(21) Anmeldenummer: **88103785.7**

(22) Anmeldetag: **10.03.88**

(51) Int. Cl.4: **C07D 213/20**, C07D 233/60, C07C 141/02

(30) Priorität: **20.03.87 DE 3709216**

(43) Veröffentlichungstag der Anmeldung: **21.09.88 Patentblatt 88/38**

(84) Benannte Vertragsstaaten: **CH DE FR GB IT LI**

(71) Anmelder: **BASF Aktiengesellschaft**
**Carl-Bosch-Strasse 38**
**D-6700 Ludwigshafen(DE)**

(72) Erfinder: **Braun, Gerold, Dr.**
**Limesstrasse 3**
**D-6700 Ludwigshafen(DE)**
Erfinder: **Tschang, Chung-Ji, Dr.**
**Hinterbergstrasse 31**
**D-6702 Bad Duerkheim(DE)**
Erfinder: **Vamvakaris, Christos, Dr.**
**Riedweg 6**
**D-6701 Kallstadt(DE)**
Erfinder: **Glaser, Klaus**
**Neuweg 14**
**D-6704 Mutterstadt(DE)**

## (54) Verfahren zur Herstellung von Sulfatobetainen.

(57) Die Erfindung betrifft ein Verfahren zur Herstellung von Sulfatobetainen durch Umsetzung einer Additionsverbindung aus einer Base mit einem tertiären N-Atom und Schwefeltrioxid mit einem Alkylenoxid in Gegenwart eines Alkylencarbonats als Lösungsmittel.

EP 0 282 908 A2

Xerox Copy Centre

## Verfahren zur Herstellung von Sulfatobetainen

Die Erfindung betrifft ein Verfahren zur Herstellung von Sulfatobetainen durch Umsetzung einer Additionsverbindung aus einer Base mit einem tertiären N-Atom und Schwefeltrioxid mit einem Alkylenoxid in Gegenwart eines Alkylencarbonats als Lösungsmittel.

In der US-PS 3 274 204 wird die Herstellung von Sulfatobetainen durch Umsetzung eines Epoxids mit einem Komplex aus einem tertiären Amin und Schwefeltrioxid, durch Umsetzung eines cyclischen Sulfats mit einem tertiären Amin oder durch Umsetzung eines Epoxids mit dem Komplex aus Dioxan und Schwefeltrioxid und anschließender Umsetzung mit einem tertiären Amin beschrieben.

Diese Verfahren werden zweckmäßigerweise, wie aus der Beschreibung und den Ausführungsbeispielen hervorgeht, in Gegenwart eines inerten Lösungsmittels, wie Toluol, Hexan, Ethylendichlorid, die unter Umständen toxikologisch nicht ganz unbedenklich sind, durchgeführt. Im übrigen hat sich gezeigt, daß die beschriebenen Verfahren im Hinblick auf Ausbeute und Reinheit der erhaltenen Produkte nicht immer befriedigen. Insbesondere bei der Umsetzung eines Komplexes aus einem tertiären Amin und Schwefeltrioxid mit Ethylenoxid in Ethylendichlorid als in der genannten US-PS in den Ausführungsbeispielen bevorzugt verwendetes Lösungsmittel können Nebenreaktionen ablaufen, die zu Verunreinigungen und zu Verfärbungen führen. Bei der Verwendung von Hexan als inertes Lösungsmittel sind die Amin-$SO_3$-Komplexe häufig nicht genügend löslich, so daß die Reaktion zum gewünschten Sulfatobetain nur sehr schleppend verläuft. Diese Nachteile treffen in gleicher Weise auch für das in der DE-OS 19 06 851 beschriebene Verfahren zu.

Aus der DE-AS 11 91 652 geht hervor, daß beispielsweise Pyridiniumethylsulfat (2-Pyridinium-1-sulfatoethan) in aufwendiger Weise durch Umsetzung von Hydroxiethylpyridiniumchlorid, erhalten aus Pyridin und Ethylenchlorhydrin, mit Chlorsulfonsäure hergestellt werden kann. Auch bei dieser Arbeitsweise lassen Ausbeute und Reinheit zu wünschen übrig.

In der deutschen Patentanmeldung P 36 35 230.6 wird vorgeschlagen, den Komplex aus tertiärem Amin und Schwefeltrioxid anstelle eines Alkylenoxids mit einem Alkylencarbonat als Alkylierungsmittel umzusetzen. Dabei müssen relativ hohe Temperaturen von 80 bis 220°C angewandt werden.

Um die Umsetzung im unteren Bereich der angegebenen Temperaturen durchführen zu können, muß die Umsetzung unter Basenkatalyse durchgeführt werden. Je nieder die Temperatur, eine um so größere Menge an Basen als Katalysatoren sind erforderlich. Vorteilhaft ist, daß bei diesen Umsetzungen kein freies Alkylenoxid auftritt. Jedoch muß, um Nebenreaktionen und Verfärbungen des Endproduktes zu vermeiden, im Verlaufe der Reaktion das Verschwinden der eingesetzten $SO_3$-Additionsverbindung, d.h. ihre Umsetzung, genau verfolgt werden.

Es besteht daher nach wie vor die Aufgabe, ein möglichst einfaches und wenig umständliches Verfahren für die großtechnische Herstellung von Sulfatobetainen zur Verfügung zu stellen.

Die Lösung der Aufgabe besteht in einem Verfahren zur Herstellung von Sulfatobetainen der Formel I

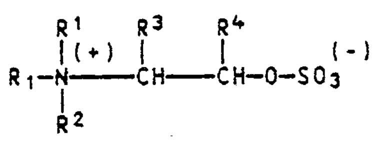

(I)

in der die einzelnen Reste $R^1$ und $R^2$ gleich oder verschieden sein können und einen gesättigten geradkettigen oder verzweigten Alkylrest mit 1 bis 22 C-Atomen, einen Cycloalkylrest mit 5 bis 7 C-Atomen im Ring, einen Phenyl-oder Naphthylrest oder einen Aralkylrest mit insgesamt 7 bis 12 C-Atomen und darüber hinaus die beiden Reste $R^1$ zusammen mit dem N-Atom einen 5-oder 6-gliedrigen heterocyclischen Ring, der gegebenenfalls ein oder mehrere weitere Heteroatome und gegebenenfalls einen annelierten Benzolring enthält und wobei für $R^2$ ein Alkylrest mit 1 bis 4 C-Atomen steht, oder die beiden Reste $R^1$ zusammen mit dem N-Atom und dem Rest $R^2$ einen 5-oder 6-gliedrigen ungesättigten heterocyclischen Ring, der gegebenenfalls ein oder mehrere weitere Heteroatome und gegebenenfalls einen annelierten Benzolring enthält, $R^3$ und $R^4$ gleich oder verschieden sind und $R^3$ ein Wasserstoffatom, einen Alkylrest mit 1 bis 7 C-Atomen, den Rest $-CH_2Cl$ oder einen Rest $-CH_2-OR^5$, wobei $R^5$ einen geradkettigen oder verzweigten Alkylrest mit 1-bis 18-C-Atomen oder Phenyl darstellt, und $R^4$ ein Wasserstoffatom, einen Alkylrest mit 1 bis 20 C-Atomen oder einen Phenylrest, wobei die Reste $R^3$ und $R^4$ miteinander vertauscht sein können, bedeuten, aus Additionsverbindungen von Basen mit einem tertiären N-Atom und Schwefeltrioxid, das dadurch gekennzeichnet ist, daß man ein Amin-$SO_3$-Addukt der Formel II

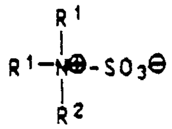

(II),

in der $R^1$ und $R^2$ die für Formel I angegebenen Bedeutungen aufweisen mit einem Alkylenoxid der Formel III

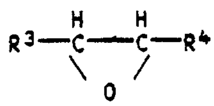

(III),

in der $R^3$ und $R^4$ die für die Formel I angegebenen Bedeutungen aufweisen, bei Temperaturen von 40 bis 150°C, bevorzugt 60 bis 90°C, in Gegenwart von Ethylencarbonat oder 1,2-Propylencarbonat oder ihren Mischungen als inerte Lösungsmittel umsetzt.

Das erfindungsgemäße Verfahren liefert in überraschender Einfachheit Sulfatobetaine in hervorragenden Ausbeuten und reine Endprodukte, die als kristallweiße Verbindungen während der Reaktion auskristallisieren. Chlorierte Kohlenwasserstoffe und andere Lösungsmittel, in denen häufig eine vollständige Lösung des Ausgangskomplexes nicht erreicht werden kann, so daß in einer Aufschlämmung gearbeitet wird, können vermieden werden. Es wird in tatsächlicher Lösung gearbeitet und gegenüber dem Verfahren der Patentanmeldung P 36 35 230.6 ist keine Basenkatalyse und genaue Reaktionskontrolle erforderlich und kann auch bei niederen Temperaturen gearbeitet werden.

Es war nicht zu erwarten, daß unter den angewandten Bedingungen das Ethylencarbonat oder 1,2-Propylencarbonat ein stabiles Lösungsmittel, das nicht in die Reaktion eingeht, darstellt. In diesen erfindungsgemäß zu verwendenden Lösungsmitteln kann vorteilhafterweise und ohne Komplikation der Amin-$SO_3$-Komplex der Formel II direkt hergestellt werden.

Im einzelnen wird zu dem erfindungsgemäßen Verfahren erläutert:

Beispielhaft zu nennende tertiäre Amine als Ausgangsverbindungen für die Amin-$SO_3$-Additionsverbindungen der Formel II sind Tridodecyl-, Tristearyl-, Tricyclohexyl-, Triphenyl-, Dimethyl-dodecyl-, Diethylphenyl-, Dimethyl-stearyl-, Trimethyl-, Triethyl-oder Tributylamin.

Zu den bevorzugten tertiären Alkylaminen gehören tertiäre Amine mit Alkylresten mit 1 bis 4 C-Atomen für jeweils $R^1$ und 12 bis 18 C-Atomen für $R^2$.

Bevorzugt sind Additionsverbindungen des Schwefeltrioxids an heterocyclische Verbindungen. Hervorzuhebende heterocyclische Verbindungen sind beispielsweise N-Methyl-und N-Ethylpiperidin, N-Methyl-und N-Ethylmorpholin, N-Methylpyrazol, Oxazol, Isoxazol, Acridin, Phenacridin, Pyrazin und Pyridazin.

Besonders bevorzugt sind Pyridin, ggfs. durch ein oder zwei Alkylreste mit 1 bis 4 C-Atomen, eine Carboxyalkylgruppe mit 1 bis 4 C-Atomen im Alkyl oder eine Nitrilgruppe substituiert, wie $\alpha$-, $\beta$-, $\gamma$-Picolin, 2-Ethylpyridin, Nicotinsäuremethylester oder Nicotinsäurenitril, Chinolin oder Isochinolin, ggfs. durch einen Methylrest substituiert, wie Chinaldin, und N-($C_1$-bis $C_{12}$-Alkyl)-imidazole, ggfs. an einem der C-Atome durch einen Alkylrest mit 1 bis 6 C-Atomen oder Phenyl substituiert, wie N-Methyl-und N-Ethylimidazol, 1,2-Dimethylimidazol, N-Methyl-2-phenylimidazol oder 1-Dodecylimidazol.

Die Herstellung der $SO_3$-Additionsverbindungen der Formel II kann in bekannter und üblicher Weise erfolgen oder besonders vorteilhaft im erfindungsgemäß zu verwendenden Lösungsmittel.

Das Ethylencarbonat oder 1,2-Propylencarbonat bzw. ihre Mischungen werden bei der erfindungsgemäßen Umsetzung zweckmäßig in einer Menge von 20 bis 50 Gew.-%, bevorzugt 30 bis 40 Gew.-%, bezogen auf das Gewicht des Amin-$SO_3$-Komplexes, verwendet. Daraus geht hervor, daß bei den angewendeten Temperaturen in für eine technische Umsetzung vorteilhaften Weise in sehr konzentrierten Lösungen gearbeitet werden kann.

Die Epoxide der Formel III sind grundsätzlich bekannt. Sie werden zweckmäßig, bezogen auf das Amin-$SO_3$-Addukt der Formel II, in einem Molverhältnis von 1 : 1 bis 1,5, bevorzugt 1 : 1,2 bis 1,3, verwendet. D.h., daß ein 20 bis 30 mol-%iger Überschuß an Epoxid zu optimalen Ausbeuten führt.

Beispielhaft zu nennende Epoxide der Formel III sind Ethylenoxid, Propylenoxid, Butylenoxid-1,2 und -2,3, Styroloxid, n-Butylglycidylether, (2-Ethylhexyl)-glycidylether oder Epichlorhydrin.

Wie oben erwähnt, erfolgt die Umsetzung einer Verbindung der Formel II mit einem Alkylenoxid der Formel III bei Temperaturen von 40 bis 150°C, bevorzugt 60 bis 90°C.

Die Reaktion ist in der Regel je nach Temperatur innerhalb von 0,5 bis 4 Stunden beendet.

Die in der Regel kristallin anfallenden Betaine können in einfacher Weise durch Absaugen abgetrennt werden. Nach dem Waschen mit einem Lösungsmittel, wie Ethanol oder Methanol, werden praktisch analysenreine Produkte erhalten.

Die nach dem erfindungsgemäßen Verfahren hergestellten Verbindungen können als Tenside, insbesondere in Waschmitteln oder als Hilfsmittel auf dem Textilgebiet oder als Galvanisierungshilfsmittel sowie als Netzmittel, Emulgatoren oder Dispergatoren verwendet werden.

Durch das erfindungsgemäße Verfahren werden insbesondere heterocyclische Sulfatoethane, die als Galvanisierungshilfsmittel hervorragende Spitzenglanzbildner sind, leicht zugänglich. Hiervon sind beispielsweise zu nennen das Pyridiniumsulfatoethan oder das Chinoliniumsulfatoethan sowie 1-Pyridinium-2-methyl-2-sulfatoethan, 1-Pyridinium-2-ethyl-2-sulfatoethan oder 1-Pyridinium-2-methylenoxy-n-butyl-2-sulfatoethan.

Beispiel 1

1-(Pyridinium)-2-sulfato-ethan

In einem Rührkessel werden 237 Teile Pyridin und 792 Teile Ethylencarbonat bei 40°C vorgelegt. Anschließend werden 240 Teile $SO_3$ zur Herstellung der Additionsverbindung bei maximal 70°C zudosiert. Unter Kühlung werden dann bei 75°C 160 Teile Ethylenoxid zugegeben. Nach vollständiger Umsetzung des Pyridin-$SO_3$-Addukts wird auf 40°C abgekühlt. Nach dem Entfernen von überschüssigem Ethylenoxid wird das kristalline Reaktionsprodukt vom Lösemittel abgetrennt und nach Waschen mit Methanol getrocknet.
Schmelzpunkt: 203°C Ausbeute: 90 %

Beispiel 2

1-(N-Methylimidazolium)-2-sulfato-ethan

In einem Rührkessel werden 41 Teile N-Methylimidazol und 200 Teile Propylencarbonat vorgelegt. Anschließend werden bei ca. 50°C 40 Teile $SO_3$ zur Herstellung der Additionsverbindung zudosiert. Unter Kühlung werden dann bei 90°C 55 Teile Ethylenoxid zugesetzt. Nach vollständiger Umsetzung wird auf Raumtemperatur abgekühlt. Das Produkt wird durch Absaugen vom Lösemittel isoliert, mit Methanol gewaschen und getrocknet.
Schmelzpunkt: 205°C Ausbeute: 91 %

Beispiel 3

1-Pyridinium-2-phenyl-2-sulfato-ethan

In einem Rührkessel werden 250 Teile Propylencarbonat und 39 Teile Pyridin vorgelegt. Durch Zudosieren von 40 Teilen $SO_3$ wird das Pyridin-$SO_3$-Addukt erhalten. Entsprechend Beispiel 2 werden 72 Teile Styroloxid zudosiert. Nach dem Abkühlen wird das Reaktionsprodukt entsprechend Beispiel 2 isoliert.
Schmelzpunkt: 213°C Ausbeute: 85 %

Beispiel 4

1-Pyridinium-2-methylene-oxi-n-butyl-2-sulfato-ethan

Herstellung wie Beispiel 3
Es werden jedoch statt Styroloxid 90 Teile n-Butylglycidether verwendet.
Ausbeute: 80°C
Schmelzpunkt: 217°C

## Ansprüche

1. Verfahren zur Herstellung von Sulfatobetainen der Formel I

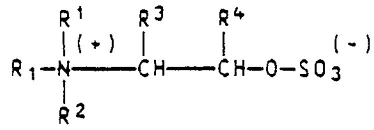

(I)

in der die einzelnen Reste $R^1$ und $R^2$ gleich oder verschieden sein können und einen gesättigten geradkettigen oder verzweigten Alkylrest mit 1 bis 22 C-Atomen, einen Cycloalkylrest mit 5 bis 7 C-Atomen im Ring, einen Phenyl-oder Naphthylrest oder einen Aralkylrest mit insgesamt 7 bis 12 C-Atomen und darüber hinaus die beiden Reste $R^1$ zusammen mit dem N-Atom einen 5-oder 6-gliedrigen heterocyclischen Ring, der gegebenenfalls ein oder mehrere weitere Heteroatome und gegebenenfalls einen annelierten Benzolring enthält und wobei für $R^2$ ein Alkylrest mit 1 bis 4 C-Atomen steht, oder die beiden Reste $R^1$ zusammen mit dem N-Atom und dem Rest $R^2$ einen 5-oder 6-gliedrigen ungesättigten heterocyclischen Ring, der gegebenenfalls ein oder mehrere weitere Heteroatome und gegebenenfalls einen annelierten Benzolring enthält, $R^3$ und $R^4$ gleich oder verschieden sind und $R^3$ ein Wasserstoffatom, einen Alkylrest mit 1 bis 7 C-Atomen, den Rest $-CH_2Cl$ oder einen Rest $-CH_2-OR^5$, wobei $R^5$ einen geradkettigen oder verzweigten Alkylrest mit 1-bis 18-C-Atomen oder Phenyl darstellt, und $R^4$ ein Wasserstoffatom, einen Alkylrest mit 1 bis 20 C-Atomen oder einen Phenylrest, wobei die Reste $R^3$ und $R^4$ miteinander vertauscht sein können, bedeuten, aus Additionsverbindungen von Basen mit einem tertiären N-Atom und Schwefeltrioxid, das dadurch gekennzeichnet ist, daß man ein Amin-$SO_3$-Addukt der Formel II

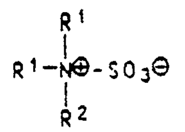

(II),

in der $R^1$ und $R^2$ die für Formel I angegebenen Bedeutungen aufweisen mit einem Alkylenoxid der Formel III

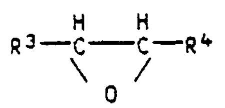

(III),

in der $R^3$ und $R^4$ die für die Formel I angegebenen Bedeutungen aufweisen, bei Temperaturen von 40 bis 150°C, in Gegenwart von Ethylencarbonat oder 1,2-Propylencarbonat oder ihren Mischungen als inerte Lösungsmittel umsetzt.

2. Verfahren nach Anspruch 1, dadurch gekennzeichnet, daß man eine heterocyclische Additionsverbindung des Schwefeltrioxids an Pyridin, das ggfs. durch ein oder zwei Alkylreste mit 1 bis 4 C-Atomen, eine Carboxyalkylgruppe mit 1 bis 4 C-Atomen im Alkyl oder eine Nitrilgruppe substituiert ist, Chinolin oder Isochinolin, die ggfs. durch eine Methylgruppe substituiert sind, oder ein N-($C_1$-bis $C_{12}$-Alkyl)-imidazol, das ggf. an einem der C-Atome durch einen Alkylrest mit 1 bis 6 Atomen oder Phenyl substituiert ist, einsetzt.

3. Verfahren nach Anspruch 1 oder 2, dadurch gekennzeichnet, daß man das Ethylencarbonat oder 1,2-Propylencarbonat oder ihre Mischungen in einer Menge von 20 bis 50 Gew.-%, bezogen auf das Amin-$SO_3$-Addukt der Formel II, einsetzt.

4. Verfahren nach einem der Ansprüche 1 bis 3, dadurch gekennzeichnet, daß man die Additionsverbindung des Schwefeltrioxids an Pyridin mit Ethylenoxid umsetzt.